(19) Europäisches Patentamt
European Patent Office
Office européen des brevets

(11) **EP 2 371 813 A1**

(12) **EUROPEAN PATENT APPLICATION**


(43) Date of publication:
**05.10.2011 Bulletin 2011/40**

(51) Int Cl.:
***C07C 255/42*** (2006.01) ***C07D 333/24*** (2006.01)
***H01L 31/04*** (2006.01) ***H01M 14/00*** (2006.01)

(21) Application number: **11250180.4**

(22) Date of filing: **16.02.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.03.2010 KR 20100026399**



(71) Applicant: **Samsung SDI Co., Ltd.**
**Gyeonggi-do (KR)**

(72) Inventors:
- **Kang, Moon-Sung**
**Yongin-si, Gyeonggi-do (JP)**
- **Lee, Ji-Won**
**Yongin-si, Gyeonggi-do (JP)**
- **Shin, Byong-Cheol**
**Yongin-si, Gyeonggi-do (JP)**
- **Ko, Jae-Jung**
**Yongin-si, Gyeonggi-do (JP)**
- **Choi, Hyun-Bong**
**Yongin-si, Gyeonggi-do (JP)**

(74) Representative: **Mounteney, Simon James**
**Marks & Clerk LLP**
**90 Long Acre**
**London**
**WC2E 9RA (GB)**


(54) **Spirobifluorene-based compound and dye-sensitized solar cell using the same**


(57) A spirobifluorene-based compound and a dye-sensitized solar cell using the spirobifluorene-based compound as a dye.


FIG. 1

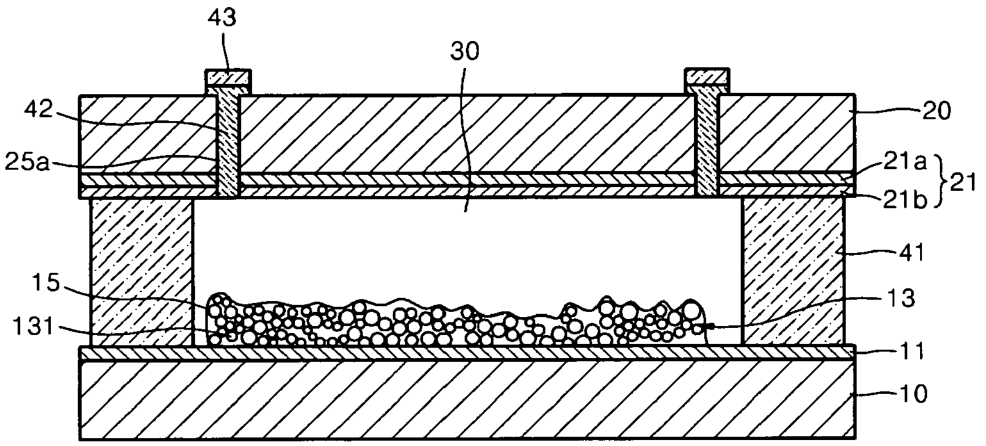





Printed by Jouve, 75001 PARIS (FR)

## Description

BACKGROUND

Field

The present invention relates to a spirobifluorene-based compound and a dye-sensitized solar cell using the same.

Description of the Related Technology

Unlike silicon solar cells, dye-sensitized solar cells are photoelectrochemical solar cells that are composed of photosensitive dye molecules, as main constituents, that may produce electron-hole pairs by absorbing visible rays, and a transition metal oxide that transfers the produced electrons. Dye-sensitized solar cells may be manufactured at lower cost than silicon-based solar cells, and since they use transparent electrodes, the cells may be applied to external glass walls of a building or glass greenhouse. However, in the past, dye-sensitized solar cells have had limited practical application due to their low photoelectric conversion efficiency.

The photoelectric conversion efficiency of a dye-sensitized solar cell is proportional to the quantity of electrons produced from the absorption of solar rays. Thus, to increase the photoelectric conversion efficiency, the quantity of the produced electrons may be increased by absorbing more sunlight or by increasing the amount of dye adsorbed, or the excited electrons so produced may be prevented from being used to cause electron-hole recombination.

To increase the adsorption amount of dye per unit area, oxide semiconductor particles need to be nano-sized, and, to increase the absorption of the sunlight, the reflectivity of a platinum electrode may be increased, or a micro-sized oxide semiconductor light scattering agent should be included to increase the absorption of solar rays. However, these conventional methods have limitations in terms of increasing the photoelectric conversion efficiency of dye-sensitized solar cells. Therefore, there is an urgent need to develop a novel method of improving the photoelectric conversion efficiency of the dye-sensitized solar cells.

SUMMARY

In a first aspect, the present invention provides a spirobifluorene-based compound according to Claim 1 of the appended set of claims. Preferred features are set out in the dependent claims. In a further aspect, the present invention provides a dye-sensitized solar cell comprising the spirobifluorene-based compound.

According to one or more embodiments of the present invention, a spirobifluorene-based compound having excellent thermal stability is provided, and a dye-sensitized solar cell using the same is provided, whereby the photoelectric conversion efficiency of the cell is enhanced.

According to the first aspect of the present invention, a spirobifluorene-based compound represented by Formula 1 below is provided.

Formula 1

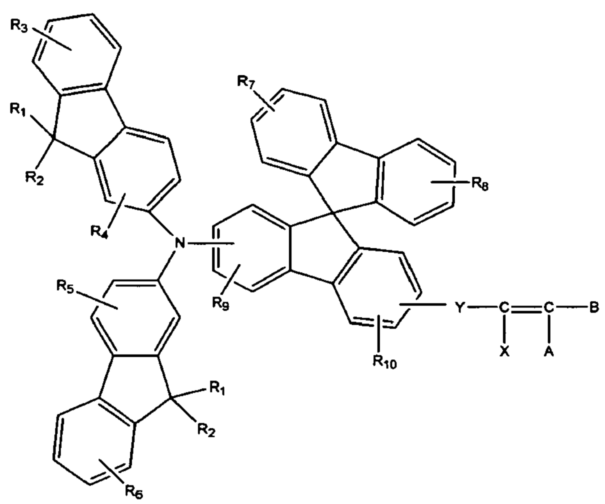

wherein Y denotes a chemical bond, or a substituted or unsubstituted $C_6$-$C_{30}$ arylene group, a substituted or unsubstituted $C_2$-$C_{30}$ heteroarylene group, or a substituted or unsubstituted $C_2$-$C_{30}$ alkynylene group,
X is hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, or a cyano group,
$R_1$ and $R_2$ are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, or a substituted or unsubstituted $C_2$-$C_{30}$ heteroaryl group,
A is a cyano group or a carboxyl group,
B is an acidic functional group, and
$R_3$ through $R_{10}$ are monosubstituted or multi-substituted groups, and are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, a substituted or unsubstituted $C_2$-$C_{30}$ alkynyl group, a substituted or unsubstituted $C_3$-$C_{30}$ carbocyclic group, a substituted or unsubstituted $C_2$-$C_{30}$ heterocyclic group, a halogen atom, a hydroxyl group, a cyano group, a thiol group, or an amino group.

According to a further aspect of the present invention, a dye-sensitized solar cell includes a first electrode, a light absorption layer formed on a surface of the first electrode, a second electrode disposed to face the first electrode on which the light absorption layer is formed, and an electrolyte disposed between the first electrode and the second electrode; and a spirobifluorene-based compound according to the present invention, wherein the spirobifluorene-based compound is used as a dye.

BRIEF DESCRIPTION OF THE DRAWINGS

These and/or other aspects of the present invention will become apparent and more readily appreciated from the following description of the embodiments, taken in conjunction with the accompanying drawings of which:

FIG. 1 is a cross-sectional view illustrating a structure of a dye-sensitized solar cell according to an embodiment;

FIG. 2 is a graph showing variation in incident photon to current efficiency (IPCE) with respect to unit wavelength of dye-sensitized solar cells manufactured according to Preparation Examples 1 through 3; and

FIG. 3 is a graph showing UV-photoluminescence (PL) characteristics of compounds represented by Formulae 4 to 6, prepared according to Synthesis Examples 1 through 3.

DETAILED DESCRIPTION

Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain aspects of the present description.

According to an embodiment, there is provided a spirobifluorene-based compound represented by Formula 1 below:

Formula 1

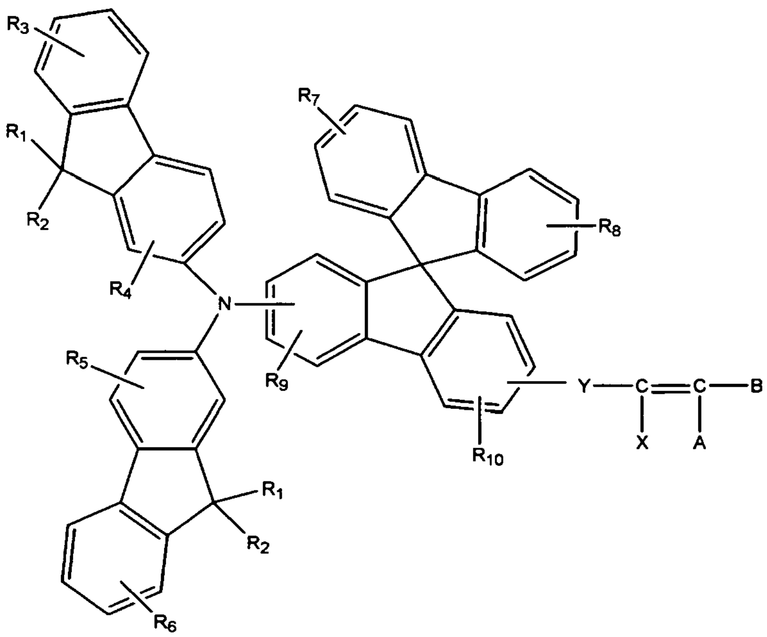

wherein Y denotes a chemical bond, or a substituted or unsubstituted $C_6$-$C_{30}$ arylene group, a substituted or unsubstituted $C_2$-$C_{30}$ heteroarylene group, or a substituted or unsubstituted $C_2$-$C_{30}$ alkynylene group,
X is hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, or a cyano group,
$R_1$ and $R_2$ are each independently hydrogen, a substituted or unsubstituted $C_1$-$C_{30}$ alkyl group, a substituted or unsubstituted $C_1$-$C_{10}$ alkoxy group, a substituted or unsubstituted $C_6$-$C_{30}$ aryl group, or a substituted or unsubstituted $C_2$-$C_{30}$ heteroaryl group,
A is a cyano group or a carboxyl group,
B is an acidic functional group, and
$R_3$ through $R_{10}$ are monosubstituted or multi-substituted groups, and are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, a substituted or unsubstituted $C_2$-$C_{30}$ alkynyl group, a substituted or unsubstituted $C_3$-$C_{30}$ carbocyclic group, a substituted or unsubstituted $C_2$-$C_{30}$ heterocyclic group, a halogen atom, a hydroxyl group, a cyano group, a thiol group, or an amino group.

The spirobifluorene-based compound of Formula 1 may be a compound represented by Formula 2 below:

Formula 2

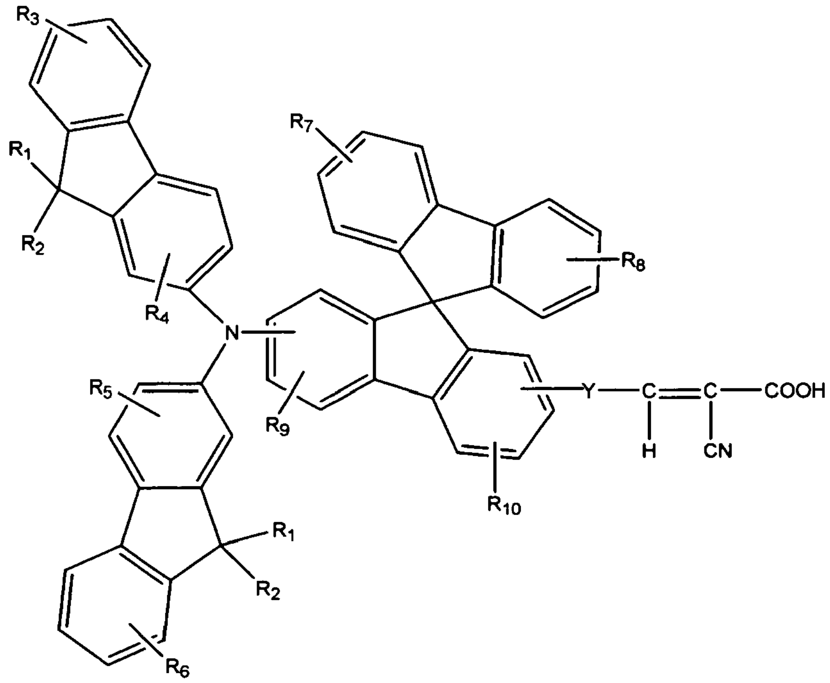

wherein Y denotes a chemical bond, or a substituted or unsubstituted $C_6$-$C_{30}$ arylene group, a substituted or unsubstituted $C_2$-$C_{30}$ heteroarylene group, or a substituted or unsubstituted $C_2$-$C_{30}$ alkynylene group,

$R_1$ and $R_2$ are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, OR a substituted or unsubstituted $C_2$-$C_{30}$ heteroaryl group, and

$R_3$ through $R_{10}$ are mono-substituted or multi-substituted groups, and are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, a substituted or unsubstituted $C_2$-$C_{30}$ alkynyl group, a substituted or unsubstituted $C_3$-$C_{30}$ carbocyclic group, a substituted or unsubstituted $C_2$-$C_{30}$ heterocyclic group, a halogen atom, a hydroxyl group, a cyano group, a thiol group, or an amino group.

The spirobifluorene-based compound of Formula 1 includes spirobifluorene having excellent thermal stability as a spacer. While not wishing to be bound to a particular theory, it is believed that this allows the compound to absorb more light with long wavelengths. The spirobifluorene activates charge separation between an electron donor ligand and an electron acceptor ligand and prevents overlapping between molecules, whereby a dye-sensitized solar cell using the compound of Formula 1 has a high open-circuit voltage. For example, the open-circuit voltage may be from about 0.6 to about 1.0 V.

The spirobifluorene-based compound of Formula 1 may be a compound represented by Formula 3 below:

Formula 3

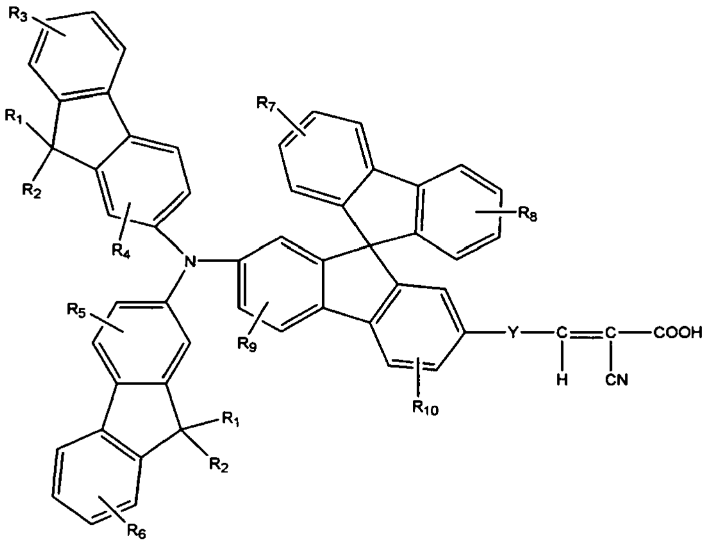

wherein Y denotes a chemical bond, or a substituted or unsubstituted $C_6$-$C_{30}$ arylene group, a substituted or unsubstituted $C_2$-$C_{30}$ heteroarylene group, or a substituted or unsubstituted $C_2$-$C_{30}$ alkynylene group,
$R_1$ and $R_2$ are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, or a substituted or unsubstituted $C_2$-$C_{30}$ heteroaryl group, and
$R_3$ through $R_{10}$ are mono-substituted or multi-substituted groups, and are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, a substituted or unsubstituted $C_2$-$C_{30}$ alkynyl group, a substituted or unsubstituted $C_3$-$C_{30}$ carbocyclic group, a substituted or unsubstituted $C_2$-$C_{30}$ heterocyclic group, a halogen atom, a hydroxyl group, a cyano group, a thiol group, or an amino group.

Y denotes a single bond, a $C_6$-$C_{20}$ arylene group, or a $C_2$-$C_{20}$ heteroarylene group. For example, Y may be selected from the groups represented by the following formulae:

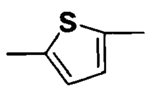 , 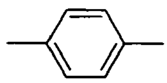

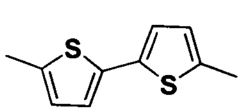 , 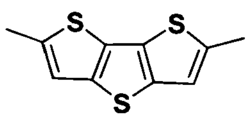 , 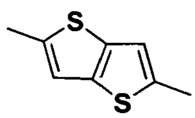 , 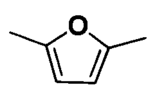 ,

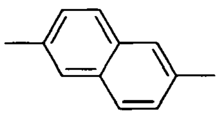 , 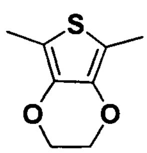 , 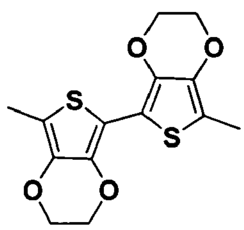

As described above, when Y is a phenylene group or thiophene group, the compound of Formula 1 absorbs more light with long wavelengths.

B of formula 1 is at least one selected from the group consisting of a carboxyl group, a phosphonic acid group, a sulfonic acid, a phosphinic acid group, an oxycarboxylic acid group, a boric acid group, and a squaric acid group. For example, B may be a carboxyl group (-COOH).

The spirobifluorene-based compound of Formula 1 may be one of the compounds represented by Formulae 4 through 6 below:

**Formula 4**

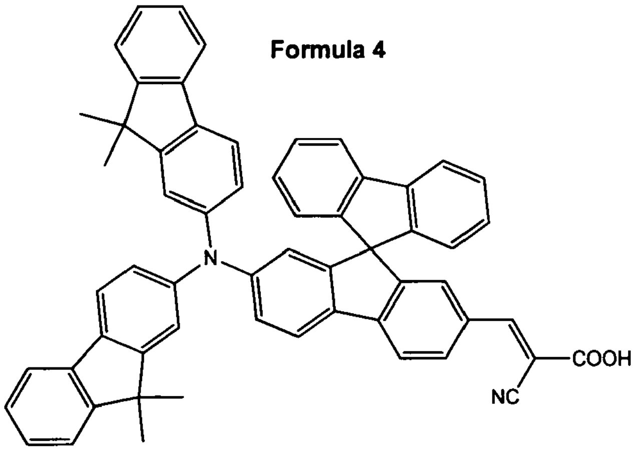

**Formula 5**

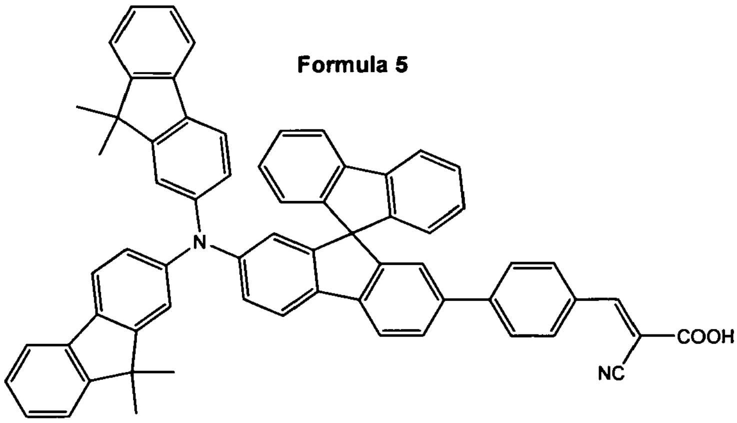

**Formula 6**

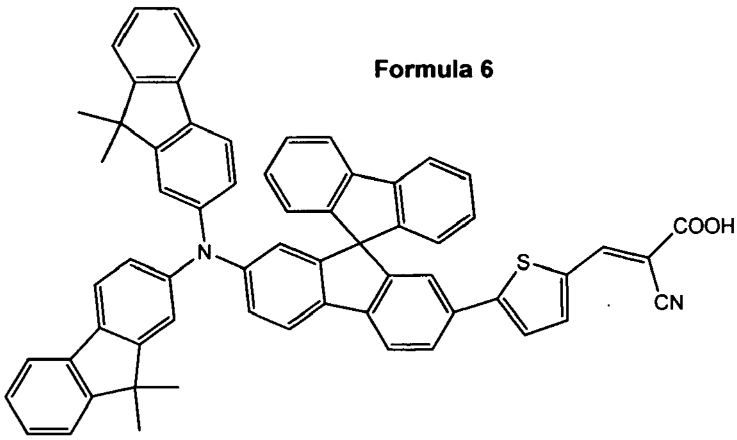

The spirobifluorene-based compound of Formula 1 may be prepared as follows.

The spirobifluorene-based compound of Formula 1 may be synthesized by reacting a compound represented

by Formula 7 below and a compound represented by Formula 8 below:

Formula 7

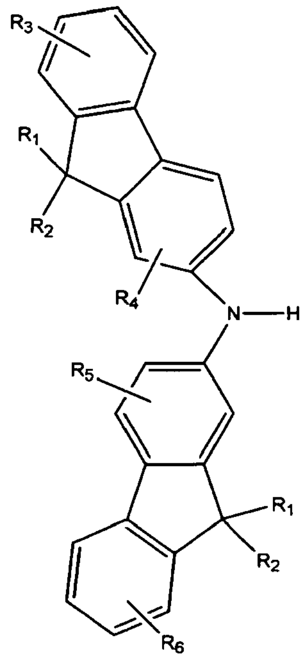

Formula 8

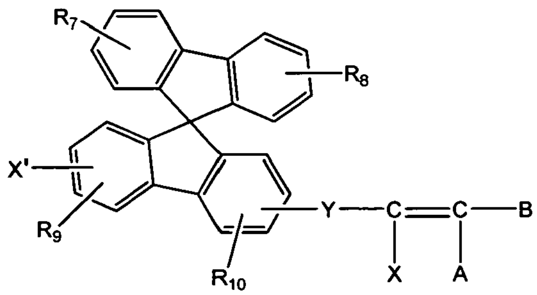

In Formula 8, Y denotes a chemical bond, or a substituted or unsubstituted $C_6$-$C_{30}$ arylene group, a substituted or unsubstituted $C_2$-$C_{30}$ heteroarylene group, or a substituted or unsubstituted $C_2$-$C_{30}$ alkynylene group,
X is hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, or a cyano group,
$R_1$ and $R_2$ are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, or a substituted or unsubstituted $C_2$-$C_{30}$ heteroaryl group,
A is a cyano group or a carboxyl group,
B is an acidic functional group, and
$R_3$ through $R_{10}$ are mono-substituted or multi-substituted groups, and are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, a substituted or unsubstituted $C_2$-$C_{30}$ alkynyl group, a substituted or unsubstituted $C_3$-$C_{30}$ carbocyclic group, a substituted or unsubstituted $C_2$-$C_{30}$ heterocyclic group, a halogen atom, a hydroxyl group, a cyano group, a thiol group, or an amino group,
X' is a halogen atom.

The halogen atom may be, for example, iodine (I), fluorine (F), bromine (Br), or chlorine (Cl).

In some embodiments, the reaction between the compound of Formula 7 and the compound of Formula 8 may

be performed by adding palladium acetate, tertiary butylphosphine, and cesium carbonate to a mixture of the compound of Formula 7 and the compound of Formula 8, adding toluene as a solvent to the resulting mixture, and then refluxing the resultant mixture.

The spirobifluorene-based compound of Formula 1 may be used as a dye for a dye-sensitized solar cell.

FIG. 1 is a cross-sectional view illustrating a structure of a dye-sensitized solar cell according to an embodiment.

Referring to FIG. 1, the dye-sensitized solar cell according to the present embodiment includes a first substrate 10 on which a first electrode 11, a photoelectrode 13, and a dye 15 are formed, a second substrate 20 on which a second electrode 21 is formed, and an electrolyte 30 disposed between the first electrode 11 and the second electrode 21 such that the first substrate 10 and the second substrate 20 face each other. A case (not shown) may be disposed at an outer side of the first substrate 10 and the second substrate 20. The structure of the dye-sensitized solar cell will now be described in more detail.

In the present embodiment, the first substrate 10, which supports the first electrode 11, may be transparent to allow external light to be incident on the first substrate 10. Thus, the first substrate 10 may be formed of glass or plastic. The plastic may be polyethylene terephthalate (PET), polyethylene naphthalate (PEN), polycarbonate (PC), polypropylene (PP), polyimide (PI), triacetyl cellulose (TAC), or the like.

The first electrode 11 formed on the first substrate 10 may be formed of a transparent material such as ZnO-$Ga_2O_3$, ZnO-$Al_2O_3$, at least one selected from indium tin oxide, indium oxide, tin oxide, zinc oxide, sulfur oxide, fluorine oxide, and mixtures thereof, or the like. The first electrode 11 may be in the form of a single film or laminated film formed of the transparent material.

The photoelectrode 13 is formed on the first electrode 11. The photoelectrode 13 includes titanium oxide particles 131, and has an appropriate average pore size, thereby easily transferring the electrolyte 30.

The thickness of the photoelectrode 13 may be from about 10 to 3000 nm, for example, from about 10 to about 1000 nm. However, the present embodiments are not limited thereto, and the thickness of the photoelectrode 13 may vary according to technology advancement, and the like.

The dye 15 that absorbs external light to produce excited electrons is adsorbed onto a surface of the photoelectrode 13. The dye 15 may be the spirobifluorene-based compound of Formula 1. For example, one of the compounds of Formula 4 through 6 may be used as the dye 15.

The second substrate 20 disposed to face the first substrate 10 supports the second electrode 21, and may be transparent. Thus, the second substrate 20 may be formed of glass or plastic, as is the first substrate 10.

The second electrode 21 formed on the second substrate 20 is disposed to face the first electrode 11, and may include a transparent electrode 21a and a catalyst electrode 21b.

The transparent electrode 21a may be formed of a transparent material such as indium tin oxide, fluoro tin oxide, antimony tin oxide, zinc oxide, tin oxide, ZnO-$Ga_2O_3$, ZnO-$Al_2O_3$, or the like. The transparent electrode 21 may be in the form of a single film or a laminated film formed of the transparent material.

The catalyst electrode 21 b activates redox couples, and may be a platinum electrode.

The first substrate 10 and the second substrate 20 are attached to each other using an adhesive 41. The electrolyte 30 is injected into the interior between the first electrode 11 and the second electrode 21 through holes 25a that penetrate the second substrate 20 and the second electrode 21. The electrolyte 30 is uniformly diffused into the photoelectrode 13. The electrolyte 30 receives electrons from the second electrode 21 and transfers the electrons to the dye 15 through reduction and oxidation. The holes 25a penetrating the second substrate 20 and the second electrode 21 are sealed by an adhesive 42 and a cover glass 43.

Although not illustrated in FIG. 1, a metal oxide film, which is a general porous film, may be further formed between the first electrode 11 and the photoelectrode 13. In this regard, the photoelectrode 13 acts as a light scattering electrode and is capable of adsorbing a large amount of dye, thereby addressing the disadvantages of conventional light scattering electrodes. Accordingly, the dye-sensitized solar cell may have high efficiency.

The porous film may be formed of metal oxide particles, and examples of the metal oxide may include titanium oxide, zinc oxide, tin oxide, strontium oxide, indium oxide, iridium oxide, lanthanum oxide, vanadium oxide, molybdenum oxide, tungsten oxide, niobium oxide, magnesium oxide, aluminum oxide, yttrium oxide, scandium oxide, samarium oxide, gallium oxide, and strontium titanium oxide. The metal oxide particles may be $TiO_2$ particles, $SnO_2$ particles, $WO_3$ particles, ZnO particles, or complexes thereof.

The substituents in formulae 1 through 8 are defined as follows.

The alkyl group used herein is in a linear or branched form, and may be methyl, ethyl, propyl, iso-butyl, sec-butyl, pentyl, iso-amyl, hexyl, heptyl, octyl, nonanyl, dodecyl, or the like. At least one hydrogen atom of the alkyl group may be substituted with a deuterium atom, a halogen atom, a hydroxyl group, a nitro group, a cyano group, an amino group, an amidino group, hydrazine, hydrazone, a carboxyl group or salts thereof, a sulfonic acid group or salts thereof, a phosphoric acid group or salts thereof, a $C_1$-$C_{10}$ alkyl group, a $C_1$-$C_{10}$ alkoxy group, a $C_2$-$C_{10}$ alkenyl group, a $C_2$-$C_{10}$ alkynyl group, a $C_6$-$C_{16}$ aryl group, or a $C_4$-$C_{16}$ heteroaryl group.

The alkoxy group used herein may be a group represented by -OAlk where Alk is an unsubstituted $C_1$-$C_{50}$ alkyl

group, and may be methoxy, ethoxy, propoxy, isopropyloxy, butoxy, penthoxy, or the like. At least one hydrogen atom of the alkoxy group may be substituted with the same substituent as in alkyl group described above.

The aryl group used herein refers to monocyclic or bicyclic aromatic hydrocarbon groups. The term "aryl" also refers to a group in which an aromatic group is fused to one or more cycloalkyl rings, and where the point of attachment to the spirobifluorene-based compound may be on the aromatic ring or on a cycloalkyl ring fused to the aromatic ring. Representative examples of the "aryl" group are phenyl, naphthyl, indanyl or tetrahydronaphthyl. At least one hydrogen atom of the aryl group may be substituted with the same substituents as mentioned in connection with the alkyl groups described above.

The heteroaryl group used herein refers to an aromatic organic compound which contains at least one heteroatom selected from the group consisting of nitrogen (N), oxygen (O), phosphorus (P), and sulfur (S). At least one hydrogen atom of the heteroaryl group may be replaced with the same substituents as mentioned in connection with the alkyl groups described above.

The heterocyclic group used herein refers to a cyclic group containing a heteroatom such as N, S, P, or O. At least one hydrogen atom of the heterocyclic group may be replaced with the same substituents as mentioned in connection with the alkyl groups described above.

The carbocyclic group used herein refers to a cyclic alkyl group. At least one hydrogen atom of the carbocyclic group may be replaced with the same substituents as mentioned in connection with the alkyl groups described above.

The alkylene group used herein may be methylene, ethylene, or the like. At least one hydrogen atom of the alkylene group may be replaced with the same substituents as mentioned in connection with the alkyl groups described above.

At least one hydrogen atom of the alkenylene group and the alkynylene group may be replaced with the same substituents as mentioned in connection with the alkyl groups described above.

The arylene group used herein may be phenylene, biphenylene, or the like, and at least one hydrogen atom of the arylene group may be replaced with the same substituents as mentioned in connection with the alkyl group described above.

At least one hydrogen atom of the heteroarylene group may be replaced with the same substituents as mentioned in connection with the alkyl groups described above.

At least one hydrogen atom of each of the heteroaryloxy group, the arylalkyl group, the aryloxy group, the carbocyclic alkyl group, the heterocyclic alkyl group, and the heteroarylalkyl group may be replaced with the same substituents as mentioned in connection with the alkyl groups described above.

One or more embodiments will now be described in further detail with reference to the following examples. However, these examples are for illustrative purposes only and are not intended to limit the scope of the present invention.

The compound of Formula 4 is synthesized according to Synthesis Example 1 through Reaction Scheme 1 below, and the compound of Formula 5 and the compound of Formula 6 are synthesized according to Synthesis Example 2 and Synthesis Example 3, respectively, through Reaction Scheme 2 below:

## Reaction Scheme 1

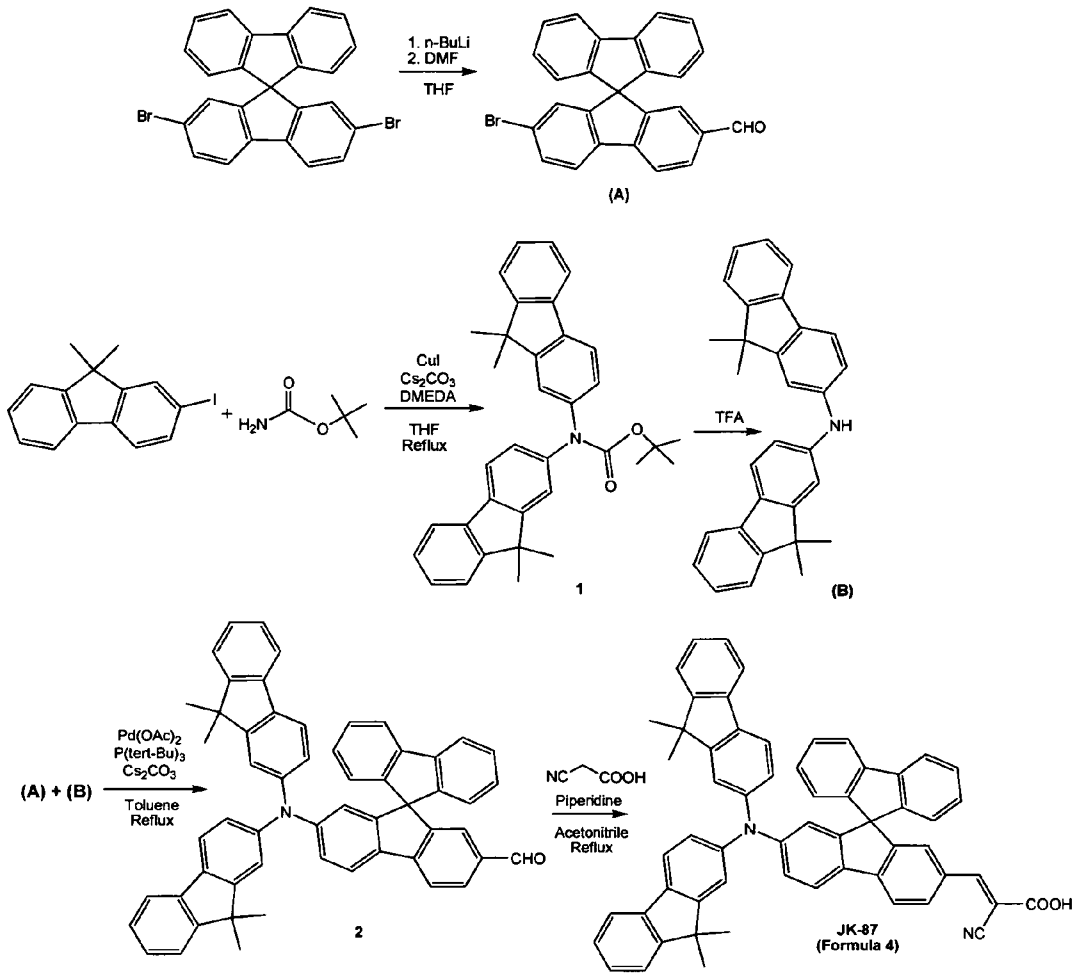

## Reaction Scheme 2

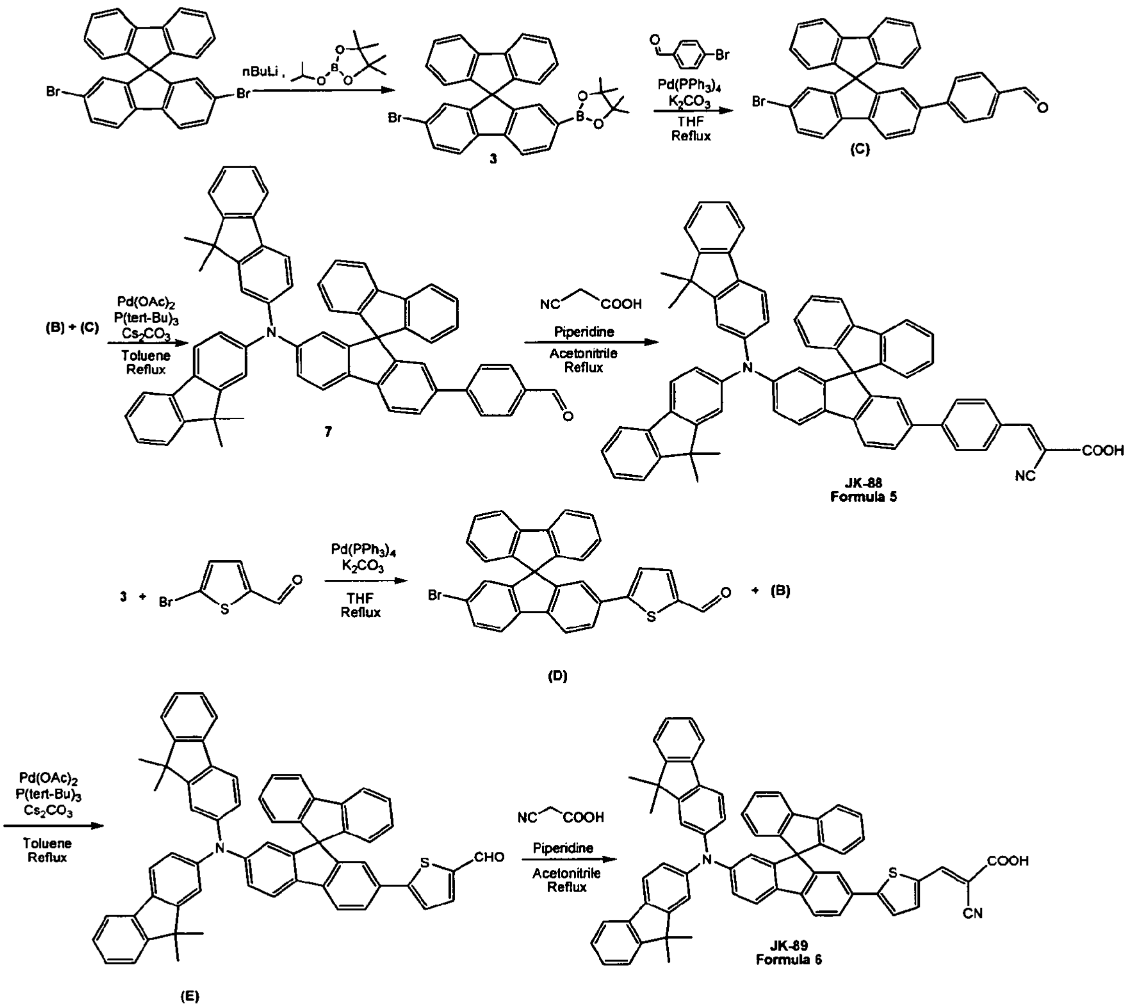


Synthesis Example 1: Preparation of compound of Formula 4 (JK-87) *tert-Butyl bis(9,9-dimethyl-9H-fluoren-7-yl)carbamate* (1)

0.014 g of copper(I) iodide (CuI(I)) (0.074 mmol), 1 g of 2-iodo-9,9-dimethylfluorene (3.12 mmol), 0.174 g of tert-butyl carbamate (1.48 mmol), and 1.45 g of $Cs_2CO_3$(4.45 mmol) were added to a flask, air was removed from the flask, and the flask was then filled with nitrogen gas. Thereafter, 0.031 ml of N,N'-dimethylethylenediamine (0.29 mmol) and 5 ml of tetrahydrofuran were added to the flask using a syringe. The flask containing the resultant mixture was refluxed at 80°C for 36 hours. After 36 hours, the temperature of the flask was reduced to room temperature, and phase separation of the resultant mixture was performed using methylene chloride, distilled water, and a separating funnel. Then, only a methylene chloride layer containing an organic material was separated from the resulting product, and the remaining moisture existing in the organic layer was removed with magnesium sulfate ($MgSO_4$). Column chromatography (stationary phase: silica gel, mobile phase: ethyl acetate:hexane=1:10 volume ratio) was performed on the resultant organic layer to obtain 0.45 g of tert-Butyl bis(9,9-dimethyl-9H-fluoren-7-yl)carbamate (1) at a yield of 60%
Mp: 156 °C
$^1H$ NMR (300 MHz, $CDCl_3$):δ 7.69-7.62 (m, 4H), 7.42-7.4 (m, 2H), 7.32 (m, 6H), 7.2-7.17 (m, 2H), 1.48 (s, 12H), 1.46 (s, 9H).
$^{13}C$ NMR (300MHz,$CDCl_3$): δ 154.27, 154.07, 153.9,142.62, 138.79, 136.61, 127.13, 125.75, 122.65, 121.42, 120.06, 119.98, 81.174, 46.99, 28.44, 27.19. Anal.cal.for $C_{35}H_{35}NO_2$: C,83.80; H,7.03; N,2.79; O,6.38.

*Bis(9,9-dimethyl-9H-fluoren-7-yl)amine (B)*

0.4 g of tert-Butoxycarbonyl-protected arylamine 1 (0.8 mmol) was dissolved in 1 ml of tetrahydrofurane, and 8 ml of trifluoroacetic acid (TFA) was added to the resulting mixture. Then, the reaction mixture was stirred at room temperature for 10 minutes, thereby obtaining a solution having a color that turned dark green. TFA was evaporated in a vacuum, dichloromethane was added to the resultant, and the resultant was neutralized using an aqueous saturated sodium hydroxide solution. The mixture was extracted with solid dichloromethane solutions as described above, and the remaining moisture in the organic layer was removed. Then, column chromatography (stationary phase: silica gel, mobile phase: ethyl acetate:hexane=1:10 volume ratio) was performed on the resultant solution to obtain 0.3 g of pale yellow Compound 2 at a yield of 95%.
Mp: 178 °C
$^1$H NMR (300 MHz, $(CD_3)_2CO$):δ 7.7-7.67 (m, 4H), 7.48 (d, *J*=6.9 Hz), 7.36 (s, 2H), 7.31-7.22 (m, 4H), 7.18 (d, 2H *J*=7.2 Hz), 1.46 (s, 12H).
$^{13}$C NMR(300MHz,$(CD_3)_2CO$):δ 155.97, 153.83, 144.45, 140.29, 132.48, 127.79, 126.73, 123.27, 121.67, 119.69, 117.32, 112.46, 47.28, 27.53.

*7-(Bis(9,9-dimethyl-9H-fluoren-7-yl)amino)-9,9-spirobifluorene-2-carbaldehyde* (2)

0.3 g of 2-bromo-7-formyl-9,9-spirobifluorene (A) (0.7 mmol), 0.44 g of Compound B (1.05 mmol), 0.023 g of palladium acetate ($Pd(OAc)_2$) (0.1 mmol), 0.04 g of tertiarybutylphosphine ($P(tBu)_3$) (0.2 mmol), and 1.4 g of cesium carbonate ($Cs_2CO_3$) (4.3 mmol) were dissolved in 15 ml of distilled toluene in a flask filled with nitrogen gas, and the mixture was refluxed at 130°C overnight. After the reaction was complete, the temperature of the flask was reduced to room temperature, and an aqueous saturated ammonium chloride solution was added to the resultant mixture. A dichloromethane layer was extracted, and the remaining moisture existing in the layer was removed with $MgSO_4$. Then, column chromatography (stationary phase: silica gel, mobile phase: dichloromethane:hexane=3:1 volume ratio) was performed on the resultant to obtain 0.26 g of a desired Compound 2 at a yield of 50%.
Mp: 180 °C
$^1$HNMR(300MHz,$(CD_3)_2CO$):δ 9.85 (s, 1H), 8.12 (d, 1H. *J*=8.4Hz),8.03(d,1H,*J*=8.4Hz),7.97(d,1H,*J*=7.8Hz),7.86(d,2H, *J*=7.2Hz),7.7(d,2H,J=7.2Hz),7 .64(d,2H,*J*==8.1 Hz),7.45(d,2H,*J*=7.2Hz),7.35(m,12H),6.96(dd,2H,*J*=8.1 Hz),6.84(d,2H, *J*=7.5 Hz),6.56(s,1H),1.28(s,12H).
$^{13}$CNMR(300MHz,$(CD_3)_2CO$):δ 191.91, 155.95, 154.39, 152.44, 150.41, 148.7, 148.53, 147.5, 142.55, 139.48, 136.41, 135.76, 135, 131.49, 129.01, 127.9, 127.66, 124.74, 124.65, 124.49, 123.41, 123.29, 121.75, 121.25, 120.74, 120.43, 119.99, 117.87, 66.52, 54.93, 47.42, 27.56.

*3-(2-(Bis(9,9-dimethyl-9H-fluoren-7-yl)amino)-9,9-spirobifluoren-7-yl)-2-cyanoacrylic acid (JK-8 7); Compound of Formula 4*

0.14 g of Compound 2 (0.188 mmol) and 0.025 g of cyanoacetatic acid (0.3 mmol) were dissolved in 15 ml of distilled chloroform, and 0.025 ml of piperidine (0.29 mmol) were added to the mixture by using a syringe. Then, the resultant mixture was refluxed for 10 hours.

After the reaction was complete, the temperature of the resultant mixture was reduced to room temperature, and a 0.1 M aqueous hydrogen chloride solution was added to the resultant mixture. The reaction mixture was extracted with chloroform, and the remaining moisture existing in the chloroform layer was removed with $MgSO_4$. Then, column chromatography (stationary phase: silica gel, mobile phase: dichloromethane:methanol=10:1 volume ratio) was performed on the resultant solution to obtain 0.068 g of the compound of Formula 4 (JK-87) at a yield of 45%.
Mp: 243 °C
$^1$H NMR (300 MHz, $(CD_3)_2SO$):δ 8.02(m, 3H), 7.83 (d, 2H *J*= 7.5 Hz), 7.75 (s, 1H), 7.67(m, 4H), 7.46 (d, 2H, *J* = 7.5 Hz), 7.33 (m, 6H), 7.16 (m, 4H), 7.02 (m, 2H), 6.85 (dd, 2H, *J* = 8.1 Hz), 6.76 (d, 2H, *J* = 7.8 Hz), 6.3 (s, 1H), 1.18 (s, 12H).
$^{13}$C NMR (300 MHz,$(CD_3)_2SO$): δ 163.32, 154.76, 153.21, 150.68, 148.83, 148.28, 147.65, 147.07, 146.1, 143.82, 141.1, 138.08, 134.31, 131.87, 129.76, 128.24, 128.11, 127.14, 126.89, 124.26, 123.6, 123.48, 122.72, 122.37, 122, 121.22, 120.6, 120.25, 119.71, 119.27, 118.8, 116.21, 111.99, 110.99, 65.31, 46.41, 26.72.

Synthesis Example 2: Preparation of compound of Formula 5 (JK-88)

Compound 3

*{2-(2-Bromo-9,9-spirobifluoren-7-yl)-4,4,5,5-tetramethyl-1,3,2-dioxaborolan}*

3.5 g of 2,7-dibromo-9,9-spirobifluorene (7.38 mmol) was dissolved in 80 ml of tetrahydrofuran in a flask filled with nitrogen gas, and the temperature of the flask was reduced to -78°C. 5.1 ml of normal-butyllithium (1.6M hexane solution) was added dropwise to the resultant mixture via a syringe. The resultant mixture was stirred at -78°C for 30 minutes, and 1.7 ml of 2-isopropoxy-4,4,5,5-tetramethyl-1,3,2-dioxaboralne (8.33 mmol) was slowly added to the resultant mixture. The reaction temperature was raised to room temperature, and the resultant mixture was then stirred for 8 hours. Water was added to the resulting mixture to complete the reaction. The reaction mixture was extracted with dichloromethane, and the remaining moisture existing in the layer was removed with $MgSO_4$. Then, column chromatography (stationary phase: silica gel, mobile phase: ethylacetate:hexane= 1:10 volume ratio) was performed on the resultant layer to obtain 3.26 g of a desired Compound 3 at a yield of 85%.
Mp: 305 °C
$^1$HNMR (300 MHz, $CDCl_3$): δ 7.85 (d, 4H *J*=7.2Hz),7.73(d,1H*J*=7.2Hz),7.48(d,1H*J*=7.2Hz),7.38(dd,2HJ=7.2Hz),7.16(s, 1H),7.11 (dd,2 H*J*=7.2Hz),6.79(s,1H),6.71(d,2H*J*=7.2Hz), 1.25(s,12H).
$^{13}$CNMR(300MHz,$CDCl_3$):δ 151.8, 147.8, 147.61, 143.8, 142, 140.4, 135.08, 134.93, 131.01, 130.41, 128.08, 128.04, 127.34, 124.29, 122.11, 121.84, 120.29, 119.51, 83.9, 65.9, 48.2, 24.9.

*4-(2-Bromo-9,9-spirobifluoren-7-yl)benzaldehyde (C)*

1.52 g of Compound 3 (2.92mmol), 0.54 g of 4-bromobenzaldehyde (2.92 mmol), 0.23 g of tetrakis(triphenylphosphine) palladium (0) {$Pd(P(C_6H_5)_3)_4$} (0.2 mmol), 4.04 g of calcium carbonate (29.2 mmol), and distilled water without oxygen were dissolved in 60 ml of distilled tetrahydrofuran in a flask filled with nitrogen gas, and the mixture was refluxed overnight. The temperature of the flask was reduced to room temperature, and the phase separation of the resultant mixture was performed using methylene chloride, distilled water, and a separatory funnel. Then, only a methylene chloride layer containing an organic material was separated from the resulting product, and the remaining moisture existing in the organic layer was removed with $MgSO_4$. Then, column chromatography (stationary phase: silica gel, mobile phase: dichloromethane:hexane=3:10 volume ratio) was performed on the resultant layer to obtain 1.23 g of desired Compound C at a yield of 85%.
Mp: 186°C.
$^1$HNMR(300MHz,$CDCl_3$):δ 9.97 (s, 1H), 7.93 (m, 3H), 7.83 (d, 2H, *J* = 8.4 Hz), 7.77 (d, 1H, $^3$J = 8.7), 7.69 (dd, 1H, *J* = 8.4 Hz), 7.58 (d, 2H, *J* = 8.7 Hz), 7.54 (dd, 1H, *J* = 8.1 Hz), 7.44 (m, 2H), 7.18 (m, 2H), 7.1(s, 1H), 6.89(s, 1H), 6.8(d, 2H, *J*= 7.8 Hz).
$^{13}$C NMR (300 MHz, $CDCl_3$): δ 191.87, 151.41, 149.67, 147.72, 146.76, 141.9, 141.25, 140.07, 139.87, 135.21, 131.24, 130.2, 128.31, 127.68, 124.22, 123, 122.09, 121.71, 120.73, 120.4, 66, 48.2.

*4-(2-(Bis(9,9-dimethyl-9H-fluoren-7-yl)amino)-9,9-spirobifluoren-7-yl)benzaldehyde (7)*

0.5 g of Compound B (1.25 mmol), 0.4 g of Compound C (0.8 mmol), 0.023 g of palladium acetate ($Pd(OAc)_2$) (0.1 mmol), 0.04 g of t-butylphosphine ($P(tBu)_3$) (0.2 mmol), and 1.4 g of cesium carbonate ($Cs_2CO_3$) (4.3 mmol) were dissolved in 15 ml of distilled toluene, and the mixture was refluxed at 130°C overnight.

After the reaction was complete, the temperature of the flask was reduced to room temperature, and an aqueous saturated ammonium chloride solution was added to the resultant mixture. A dichloromethane layer was extracted, and the remaining moisture existing in the layer was removed with $MgSO_4$. Then, column chromatography (stationary phase: silica gel, mobile phase: dichloromethane:hexane=3:1 volume ratio) was performed on the resultant to obtain 0.33 g of a desired Compound 7 at a yield of 50%.
Mp:185°C.
$^1$H NMR (300 MHz, $CDCl_3$): δ 9.97 (s, 1H), 7.9 (m, 6H), 7.69 (m, 8H), 7.41 (m, 9H), 7.2 (m, 4H), 7.03 (m, 4H), 6.75 (s, 1H), 1.33 (s, 12H).
$^{13}$C NMR (300 MHz, $CDCl_3$):δ 191.85, 155.03, 153.6, 150.7, 149.96, 148.59, 148.41, 147.04, 142.26, 141.8, 138.97, 138.29, 135.42, 134.95, 134.35, 130.15, 128.03, 127.33, 127.07, 126.6, 124.29, 123.45, 122.7, 122.54, 121.05, 120.63, 120.29, 119.87, 119.5, 118.65, 66.09, 46.83, 27.13.

*3-(4-(2-(Bis(9,9-dimethyl-9H-fluoren-7-yl)amino)-9,9-spirobifluoren-7-yl)phenyl)-2-cyanoacrylic acid (JK-88); Compound of Formula 5*

0.27 g of Compound 7 (0.33 mmol) and 0.056 g of cyanoacetic acid (0.66 mmol) were dissolved in 8 ml of distilled chloroform, 0.065 ml of piperidine (0.66 mmol) was added to the mixture via a syringe, and the resultant mixture was then refluxed for 10 hours.

After the reaction was complete, the temperature of the resultant mixture was reduced to room temperature, and a 0.1 M aqueous hydrogen chloride solution was added to the resultant mixture. A chloroform layer was extracted, and the remaining moisture existing in the layer was removed with $MgSO_4$. Then, column chromatography (stationary phase: silica gel, mobile phase: dichloromethane:methanol=10:1 volume ratio) was performed on the resultant to obtain 0.32 g of the compound JK-88 (Compound of Formula 5) at a yield of 55%.
Mp: 263°C.
$^1H$ NMR (300 MHz, $(CD_3)_2SO$):δ 7.99 (m, 3H), 7.82 (m, 4H), 7.65 (d, 2H, *J* = 6.9 Hz), 7.59 (d, 2H, *J*= 8.1 Hz), 7.51(d, 2H, *J*= 7.2 Hz), 7.43 (d, 2H, *J* = 6.3 Hz), 7.28(m, 9H), 7.12(m, 4H), 6.82(m, 4H), 6.31 (s, 1H), 1.15(s, 12H).
$^{13}C$ NMR (300 MHz, $(CD_3)_2SO$): δ 163.69, 154.77, 153.21, 150.29, 149.27, 148.06, 147.75, 147.4, 146.3, 141.89, 141.42, 141.16, 138.15, 137.73, 134.93, 134.15, 131.97, 130.17, 128.27, 128.11, 127.18, 126.84, 123.56, 123.42, 122.75, 122.34, 121.93, 121.22, 120.63, 119.71, 119.03, 118.61, 116.75, 112.43, 46.42, 26.75.

Synthesis Example 3: Preparation of compound of Formula 6 (JK-89)

*5-(2-Bromo-9,9-spirobifluoren-7-yl)thiophene-2-carbaldehyde (D)*

0.45 g of Compound 3 (0.86 mmol), 0.15 ml of 5-bromothiophene-2-carbaldehyde (1.3 mmol), 0.07 g of tetrakis (triphenylphosphine) palladium (0) {$Pd(P(C_6H_5)_3)_4$} (0.06 mmol), 1.19 g of potassium carbonate (8.61 mmol), and 4.3 ml of distilled water without oxygen were dissolved in tetrahydrofuran, and the mixture was refluxed overnight to obtain 0.34 g of Compound D at a yield of 80%.
Mp: 225°C.
$^1H$ NMR (300 MHz, $CDCl_3$):δ 9.79 (s, 1H), 7.89 (m, 3H), 7.74 (dd, 2H), 7.6 (d, 1H, *J* = 4.8 Hz), 7.53 (dd, 1H), 7.42(m, 2H), 7.19(d, 1H, *J* = 4.2 Hz), 7.14(m, 2H), 6.99(dd, 1H), 6.86(s, 1H), 6.76(dd, 2H).
$^{13}C$ NMR (300 MHz, $CDCl_3$): δ 182.64, 153.8, 151.31, 149.74, 148.13, 147.31, 142.23, 142.02, 141.79, 139.73, 137.29, 136.65, 134.68, 133.04, 131.52, 131.25, 130.48, 128.36, 127.38, 126.66, 124.07, 122.3, 121.8, 121.79, 120.87, 120.4, 83.29, 48.24.

*5-(2-(Bis(9,9-dimethyl-9H-fluoren-7-yl)amino)-9,9-spirobifluoren-7-yl)thiophene-2-carbaldehyde (E)*

1.05 g of Compound B (2.61 mmol), 0.88 g of Compound D (1.74 mmol), 0.016 g of palladium acetate ($Pd(OAc)_2$) (0.071 mmol), 0.03 g of t-butylphosphine ($P(tBu)_3$) (0.148 mmol), and 1.25 g of cesium carbonate ($Cs_2CO_3$) (3.84 mmol) were dissolved in 30 ml of distilled toluene, and the mixture was refluxed at 130°C overnight.

After the reaction was complete, the temperature of the flask was reduced to room temperature, and an aqueous saturated ammonium chloride solution was added to the resultant mixture. The resultant mixture was extracted with chloroform and the remaining moisture existing in the layer was removed with $MgSO_4$. Then, column chromatography (stationary phase: silica gel, mobile phase: dichloromethane:hexane=3:1 volume ratio) was performed on the resultant to obtain 1.15 g of a desired Compound E at a yield of 60%.
Mp: 197°C.
$^1H$ NMR (300 MHz, $CDCl_3$):δ 9.96 (s, 1H), 7.98 (m, 6H), 7.77 (dd, 3H), 7.68 (d, 2H, *J*= 7.5 Hz), 7.55(m, 8H), 7.31(dd , 6H), 7.11(m, 4H), 6,85(s, 1H), 1.46(s, 12H).
$^{13}C$ NMR (300 MHz, $CDCl_3$): δ 182.69, 155.06, 154.64, 154.64, 150.74, 150.12, 148.28, 146.97, 143.22, 141.89, 141.8, 138.97, 137.35, 135.08, 134.46, 131.63, 128.07, 127.08, 126.64, 124.16, 124.01, 123.91, 123.55, 123.28, 122.57, 121.68, 121.14, 120.65, 120.36, 119.97, 119.52, 118.74, 118.47, 65.99, 46.86, 27.14.

*3-(5-(2-Bis(9,9-dimethyl-9H-fluoren-7-yl)amino)-9,9-spirobifluoren-7-yl)thiophen-2-yl)-2-cyanoacrylic acid (JK-89); Compound of Formula 6*

0.19 g of Compound E (0.23 mmol) and 0.04 g of cyanoacetate (0.43 mmol) were dissolved in 12 ml of distilled chloroform, 0.047 ml of piperidine (0.47 mmol) was added to the mixture via a syringe, and the resultant mixture was then refluxed for 10 hours.

After the reaction was complete, the temperature of the resultant mixture was reduced to room temperature, and a 0.1 M aqueous hydrogen chloride solution was added to the resultant mixture. The reaction mixture was extracted

with chloroform, and the remaining moisture existing in the layer was removed with $MgSO_4$. Then, column chromatography (stationary phase: silica gel, mobile phase: dichloromethane:methanol=10:1 volume ratio) was performed on the resultant to obtain 0.1 g of the compound JK-89 (Compound of Formula 6) at a yield of 50%.
Mp: 285°C.
$^1H$ NMR (300 MHz, $(CD_3)_2SO$):δ 8.06 (s, 1H), 7.98 (m, 2H), 7.86 (d, 2H, *J* = 7.2 Hz), 7.79(d, 1H, *J*= 8.1 Hz), 7.67(d, 2H, *J*= 7.2 Hz), 7.62(d, 2H, *J*= 8.4 Hz), 7.45(m, 3H), 7.34(d, 1H, *J* = 7.8 Hz), 7.28(m, 7H), 7.14(m, 4H), 7.04(d, 1H, *J* = 8.4 Hz), 6.84(m, 4H), 6.3(s, 1H), 1.18(s, 12H).
$^{13}C$ NMR (300 MHz, $(CD_3)_2SO$):δ 189.11, 163.51, 154.67, 153.12, 150.24, 149.19, 148.46, 147.7, 147.84, 146.13, 142.08, 141.29, 141.01, 138.04, 135.62, 134.43, 134.11, 131.41, 128.24, 128.11, 127.04, 126.76, 126.24, 124.48, 123.47, 123.36, 122.62, 122.2, 121.92, 121.1, 120.67, 119.91, 119.91, 119.6, 118.57, 118.82, 65.34, 46.33, 26.64.

Example 1: Manufacture of dye-sensitized solar cell

A dispersion of titanium oxide particles each having a diameter of about 10 nm was coated onto a 1 $cm^2$ area of a conductive film, formed of ITO, of a first electrode by using a doctor blade. The resultant layer was heat-treated and sintered at 450°C for 30 minutes to prepare a porous film having a thickness of 10 μm.

Subsequently, the temperature of the resultant film was maintained at 80°C, and then the resultant film was impregnated in 0.3 mM of a dye dispersion in which the compound of Formula 4 was dissolved in ethanol, and a dye adsorption treatment was performed for 12 hours or more.

The dye-adsorbed porous film was cleaned using ethanol and dried at room temperature to manufacture the first electrode including a light absorption layer.

Separately, a platinum catalyst electrode was formed on the conductive film formed of ITO from above to form a second electrode. In order to facilitate injection of an electrolyte, fine holes were formed using a drill having a diameter of 0.75 mm.

A support was formed of a thermoplastic polymer film (Surlyn, DuPont, USA) having a thickness of 60 μM. It was positioned between the first electrode with the porous film formed thereon and the second electrode. Then, the resultant multi-layer structure was pressed under pressure at 100°C for 9 seconds to join the first and second electrodes together. Then, the electrolyte was injected into the interior between the first electrode and the second electrode through the fine holes formed in the second electrode. The fine holes were sealed using a cover glass and a thermoplastic polymer film to complete the manufacture of the dye-sensitized solar cell.

The electrolyte used was prepared by dissolving 0.6 M of 1,2-dimethyl-3-hexylimidazolium iodide, 0.5 M of 4-tert-butylpyrimidine, 0.1 M of LiI, and 0.05 M of $I_2$ in acetonitrile.

Example 2: Manufacture of dye-sensitized solar cell

A dye-sensitized solar cell was manufactured in the same manner as in Example 1, except that the compound of Formula 5 was used instead of the compound of Formula 4.

Example 3: Manufacture of dye-sensitized solar cell

A dye-sensitized solar cell was manufactured in the same manner as in Example 1, except that the compound of Formula 6 was used instead of the compound of Formula 4.

Comparative Example 1: Manufacture of dye-sensitized solar cell

A dye-sensitized solar cell was manufactured in the same manner as in Example 1, except that a N719 dye (Ruthenium complex dye) represented by the following formula was used instead of the compound of Formula 4:

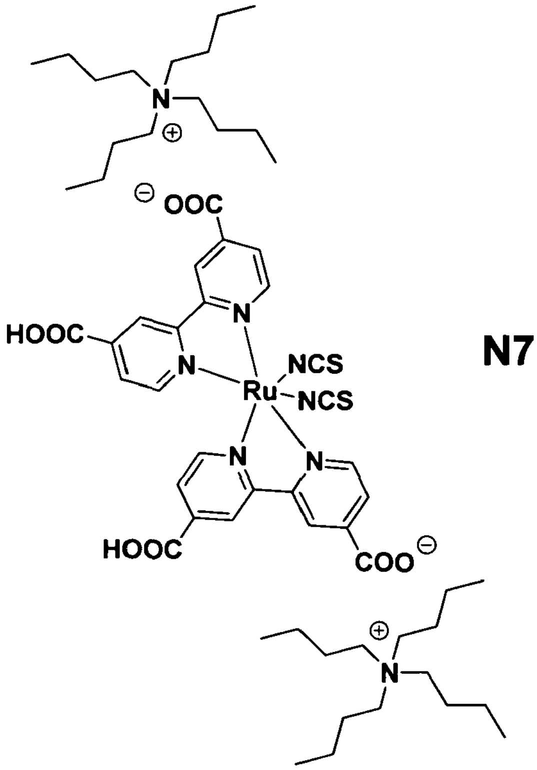

N719

The thermal stability of each of the compounds of Formulae 4 through 6 prepared according to Synthesis Examples 1, 2, and 3, respectively was evaluated by measuring a melting point of each compound.
As a result of the evaluation, it was confirmed that each compound has excellent thermal stability.
In addition, cyclic-voltammetry of each of the compounds of Formulae 4 through 6 respectively prepared according to Examples 1 through 3 was performed, and the results are shown in Table 1 below. In the Table 1, JK-87, JK-88, and JK-88 denote the compounds of Formulae 4 through 6, respectively.

Table 1

| Dye | $E_{redox}$( $\Delta E$p)$N$ | $E_{0-0}N$ | $E_{LUMO}N$ |
|---|---|---|---|
| JK-87 | 1.14 | 2.47 | -1.33 |
| JK-88 | 1.12 | 2.47 | -1.36 |
| JK-89 | 1.13 | 2.43 | -1.31 |

From the results shown in Table 1, it can be seen that lowest unoccupied molecular orbital (LUMO) potentials (LUMO energy level) of the compounds of Synthesis Examples 1 through 3 are lower (i.e., -1.33 to -1.36V (NHE basis)) than the potential of a conduction band (-0.5 V) of $TiO_2$, and thus the compounds of Synthesis Examples 1 through 3 had bands that facilitate electron injection.
In addition, highest unoccupied molecular orbital (HOMO) potentials of the spirobifluorene-based compounds of Synthesis Examples 1 through 3 are from about 1.12 to about 1.14 V (NHE basis), which are more positive values than a redox potential of $I^-/I_3^-$(i.e., 0.4 V), and thus, it is confirmed that the compounds of Synthesis Examples 1 through 3 had bands that facilitate electron regeneration.
FIG. 2 is a graph showing variation in incident photon to current efficiency (IPCE) with respect to unit wavelength of the dye-sensitized solar cells manufactured according to Examples 1 through 3. In FIG. 2, JK-87, JK-88, and JK-89 are the compounds respectively used in the dye-sensitized solar cells of Examples 1, 2, and 3.
Referring to FIG. 2, the dye-sensitized solar cells of Examples 1 through 3 absorb visible rays with a wavelength of 600 nm or greater, thereby being capable of producing electricity. In addition, each dye-sensitized solar cell maintains a photoelectric conversion efficiency of 70% or greater at a wavelength from about 360 to about 540 nm, and it is confirmed that each dye-sensitized solar cell exhibits high IPCE, which is close to about 90% while taking into consideration the reflection and absorption of glass.
UV/photoluminescence (UV/PL) characteristics of the compounds of Formulae 4 through 6 of Synthesis Examples 1 through 3 were evaluated, and the results are shown in FIG. 3 and Table 2 below. In FIG. 3, "in EtOH" represents

a case where the UV/PL characteristics are measured in an ethanol solution state, the concentration of a dye dissolved in ethanol is $3 \times 10^{-5}$ M, and "on $TiO_2$" represents UV spectra of $TiO_2$ films onto which one selected from the group consisting of the compounds of Formulae 4 through 6 as a dye are adsorbed.

Table 2

| Dye | $\lambda_{abs}$/nm ($\varepsilon$/$M^{-1}cm^{-1}$) |
|---|---|
| JK-87 | 369 (22,000) |
| | 427 (19,000) |
| JK-88 | 371 (32,000) |
| | 410 (28,000) |
| JK-89 | 368 (40,000) |
| | 428 (34,000) |

Referring to Table 2, JK-87, JK-88, and JK-89 have maximum absorption coefficients at an absorption wavelength from about 410 to 420 nm, and it is confirmed that each dye has an absorption band up to a wavelength of 500 nm. In addition, it is confirmed that the absorption coefficient of each dye is higher than that of the N719 dye (ruthenium complex dye) of Comparative Example 1 (absorption coefficient ($\varepsilon$): 13,000). Referring to FIG. 3, it is confirmed that the absorption wavelength after each dye is adsorbed onto the $TiO_2$ film is around 600 nm.

Open-circuit voltage ($V_{OC}$), current density (Jsc), energy conversion efficiency ($E_{ff}$), and fill factor (FF) of each of the dye-sensitized solar cells of Examples 1 through 3 were measured, and the results are shown in Table 3 below.

The measurement conditions of the open-circuit voltage ($V_{OC}$), current density (Jsc), energy conversion efficiency ($E_{ff}$), and fill factor (FF) in Table 3 below are as follows:

(1) Open-circuit voltage ($V_{OC}$) and current density (Jsc)
: The open-circuit voltage and the current density were measured using a Keithley SMU2400.
(2) Energy conversion efficiency ($E_{ff}$) and fill factor (FF)

The energy conversion efficiency was measured using a 1.5 AM 100 $mW/cm^2$ solar simulator (composed of an Xe lamp [300W, Oriel], AM1.5 filter, and Keithley SMU2400), and the fill factor was calculated using the obtained energy conversion efficiency given by an Equation below:
Equation

$$\text{fill factor (\%)} = \{(J \times V)_{max} / (J_{sc} \times V_{oc})\} \times 100$$

In the above Equation, J denotes a Y-axis value of an energy conversion efficiency curve, V denotes an X-axis value of the energy conversion efficiency curve, and $J_{sc}$ and $V_{oc}$ denotes intercept values of each axis.

Table 3

| Dye | $J_{SC}$($mAcm^{-2}$) | $V_{OC}$(V) | *FF* | $\eta$(%) |
|---|---|---|---|---|
| JK-87 | 11.31 | 0.78 | 0.75 | 6.58 |
| JK-8 | 9.28 | 0.76 | 0.75 | 5.28 |
| JK-89 | 13.02 | 0.75 | 0.70 | 6.83 |

From the results shown in Table 3, it is confirmed that the dye-sensitized solar cells of Examples 1 through 3 have a higher open-circuit voltage than the open-circuit voltage (0.6 to 0.7 V) of the dye-sensitized solar cell manufactured using the conventional organic dye, and have an enhanced energy conversion efficiency and fill factor.

As described above, according to one or more of the above embodiments, a dye-sensitized solar cell manufactured using a spirobifluorene-based compound having excellent thermal stability may have an enhanced photoelectric conversion efficiency.

It should be understood that the example embodiments described herein should be considered in a descriptive sense only and not for purposes of limitation. Descriptions of features or aspects within each embodiment should typically be considered as available for other similar features or aspects in other embodiments.

## Claims

1. A spirobifluorene-based compound represented by Formula 1 below:

Formula 1

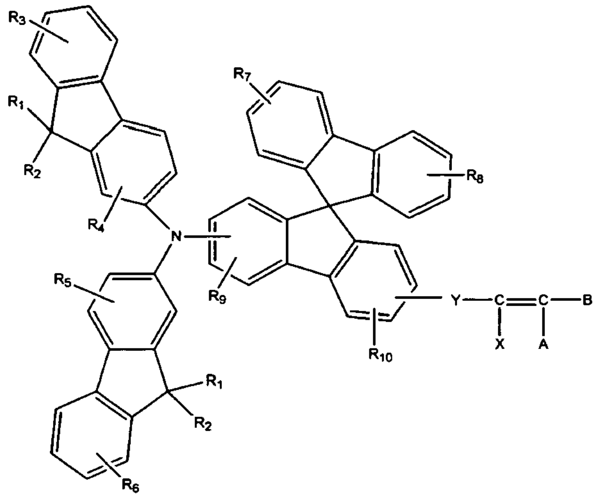

wherein Y is a chemical bond, a substituted or unsubstituted $C_6$-$C_{30}$ arylene group, a substituted or unsubstituted $C_2$-$C_{30}$ heteroarylene group, or a substituted or unsubstituted $C_2$-$C_{30}$ alkynylene group,
X is hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, or a cyano group,
$R_1$ and $R_2$ are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, or a substituted or unsubstituted $C_2$-$C_{30}$ heteroaryl group,
A is a cyano group or a carboxyl group,
B is an acidic functional group, and
$R_3$ through $R_{10}$ are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, a substituted or unsubstituted $C_2$-$C_{30}$ alkynyl group, a substituted or unsubstituted $C_3$-$C_{30}$ carbocyclic group, a substituted or unsubstituted $C_2$-$C_{30}$ heterocyclic group, a halogen atom, a hydroxyl group, a cyano group, a thiol group, or an amino group.

2. A spirobifluorene-based compound according to claim 1, wherein B is at least one selected from the group consisting of a carboxyl group, a phosphonic acid group, a sulfonic acid group, a phosphinic acid group, an oxycarboxylic acid group, a boric acid group, and a squaric acid group.

3. A spirobifluorene-based compound according to claim 2, wherein B is a carboxyl group.

4. A spirobifluorene-based compound according to any preceding claim, wherein A is a cyano group.

5. A spirobifluorene-based compound according to claim 1, wherein the spirobifluorene-based compound is a compound represented by Formula 2 below:

Formula 2

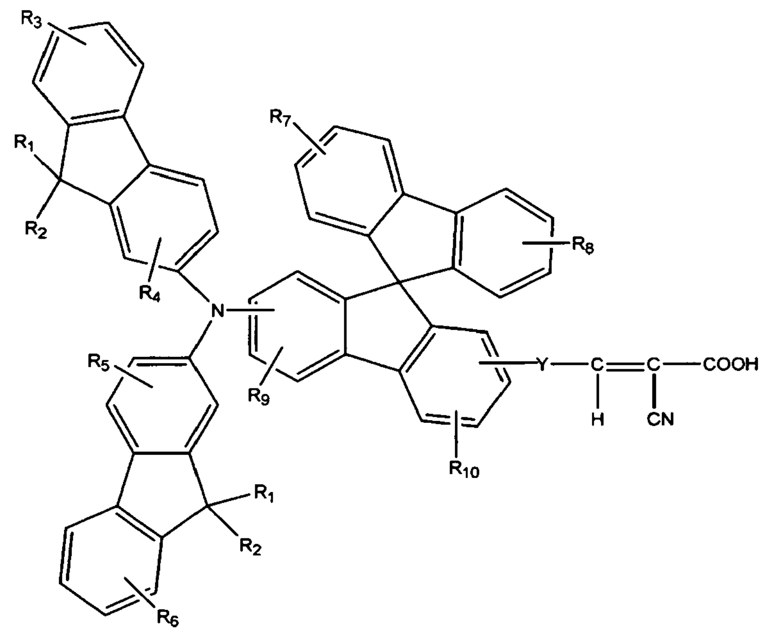

wherein Y denotes a chemical bond, or a substituted or unsubstituted $C_6$-$C_{30}$ arylene group, a substituted or unsubstituted $C_2$-$C_{30}$ heteroarylene group, or a substituted or unsubstituted $C_2$-$C_{30}$ alkynylene group,

$R_1$ and $R_2$ are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, or a substituted or unsubstituted $C_2$-$C_{30}$ heteroaryl group, and

$R_3$ through $R_{10}$ are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, a substituted or unsubstituted $C_2$-$C_{30}$ alkynyl group, a substituted or unsubstituted $C_3$-$C_{30}$ carbocyclic group, a substituted or unsubstituted $C_2$-$C_{30}$ heterocyclic group, a halogen atom, a hydroxyl group, a cyano group, a thiol group, or an amino group.

**6.** A spirobifluorene-based compound according to claim 1, wherein the spirobifluorene-based compound is a compound represented by Formula 3 below:

Formula 3

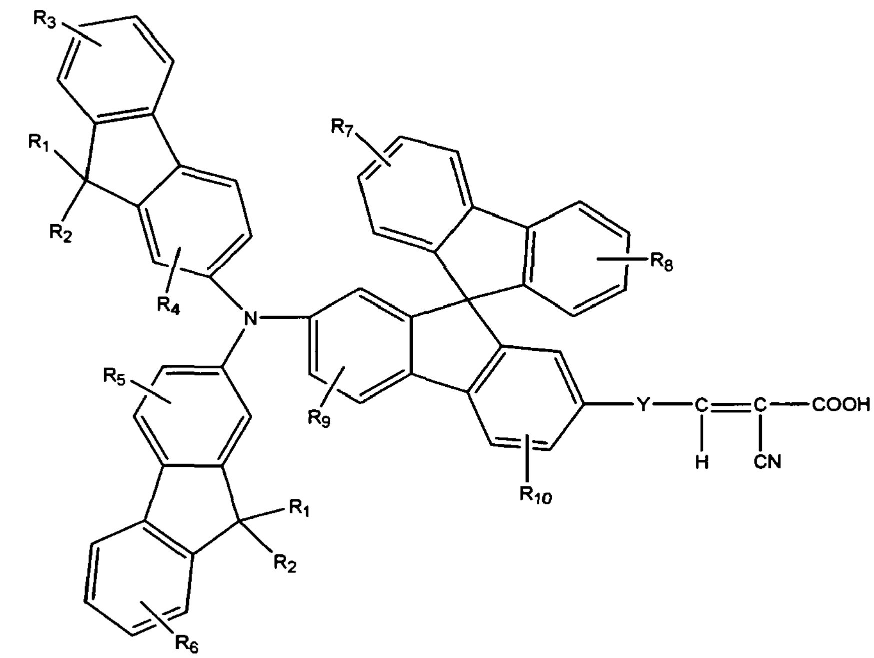

wherein Y denotes a chemical bond, or a substituted or unsubstituted $C_6$-$C_{30}$ arylene group, a substituted or unsubstituted $C_2$-$C_{30}$ heteroarylene group, or a substituted or unsubstituted $C_2$-$C_{30}$ alkynylene group,
$R_1$ and $R_2$ are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, or a substituted or unsubstituted $C_2$-$C_{30}$ heteroaryl group, and
$R_3$ through $R_{10}$ are each independently hydrogen, a substituted or unsubstituted $C_1$-$C_{30}$ alkyl group, a substituted or unsubstituted $C_1$-$C_{30}$ alkoxy group, a substituted or unsubstituted $C_6$-$C_{30}$ aryl group, a substituted or unsubstituted $C_2$-$C_{30}$ heteroaryl group, a substituted or unsubstituted $C_2$-$C_{30}$ alkynyl group, a substituted or unsubstituted $C_3$-$C_{30}$ carbocyclic group, a substituted or unsubstituted $C_2$-$C_{30}$ heterocyclic group, a halogen atom, a hydroxyl group, a cyano group, a thiol group, or an amino group.

**7.** A spirobifluorene-based compound according to any preceding claim, wherein Y is a single bond, a $C_6$-$C_{20}$ arylene group, or a $C_2$-$C_{20}$ heteroarylene group.

**8.** A spirobifluorene-based compound according to any preceding claim, wherein Y is one selected from the groups represented by the following formulae:

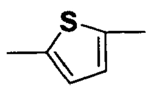 , 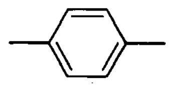

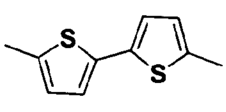 , 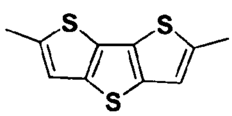 , 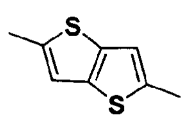 , 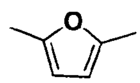 ,

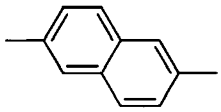 , 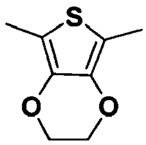 , 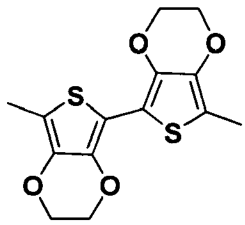

9. A spirobifluorene-based compound according to claim 8, wherein Y is one selected from the groups represented by the following formulae:

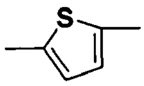 , 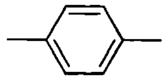 .

10. A spirobifluorene-based compound according to any preceding claim, wherein $R_1$ and $R_2$ are each hydrogen.

11. A spirobifluorene-based compound according to any preceding claim, wherein each of $R_3$ through $R_{10}$ is hydrogen.

12. A spirobifluorene-based compound according to claim 1, wherein the spirobifluorene-based compound is one selected from compounds represented by Formulae 4 through 6 below:

**Formula 4**

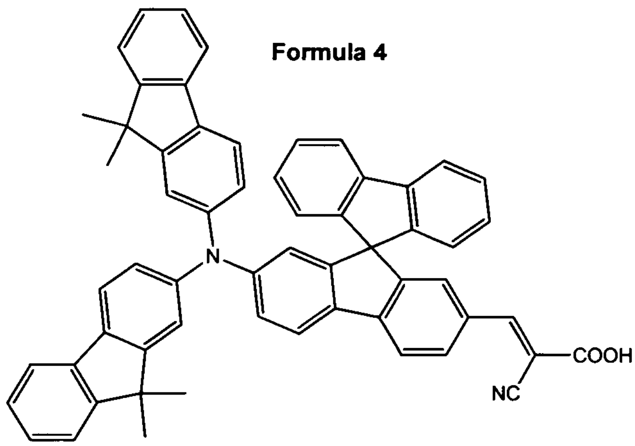

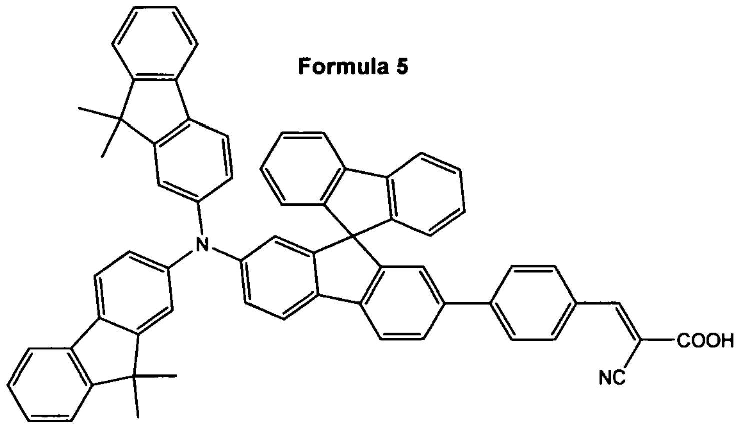

Formula 5

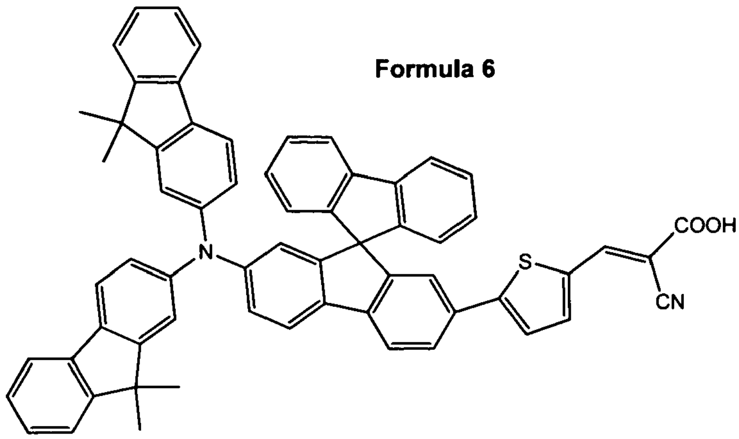

Formula 6

**13.** A spirobifluorene-based compound according to claim 1, wherein the spirobifluorene-based compound is the compound represented by Formula 4 below:

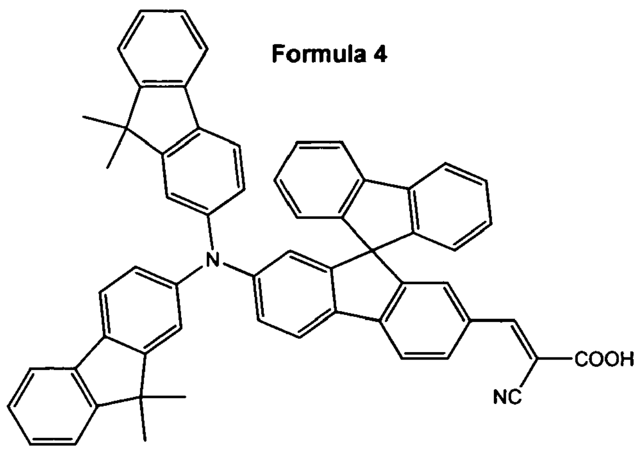

Formula 4

**14.** A spirobifluorene-based compound according to claim 1, wherein the spirobifluorene-based compound is the compound represented by Formula 5 below:

**Formula 5**

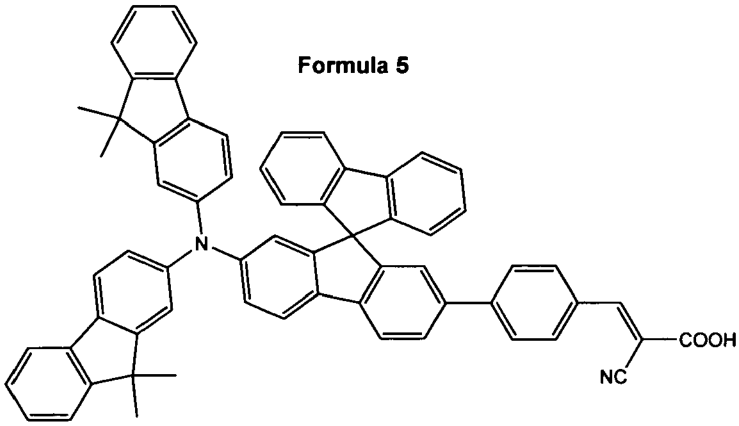

**15.** A dye-sensitized solar cell comprising:

a first electrode, a light absorption layer formed on a surface of the first electrode, a second electrode disposed to face the first electrode on which the light absorption layer is formed, and an electrolyte disposed between the first electrode and the second electrode; and
a spirobifluorene-based compound according to any of claims 1 to 14, wherein the spirobifluorene-based compound is used as a dye.

FIG. 1

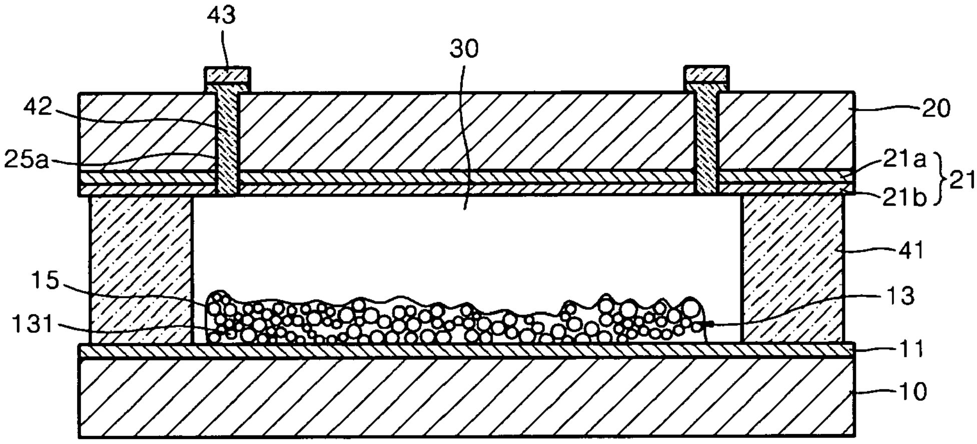

FIG. 2

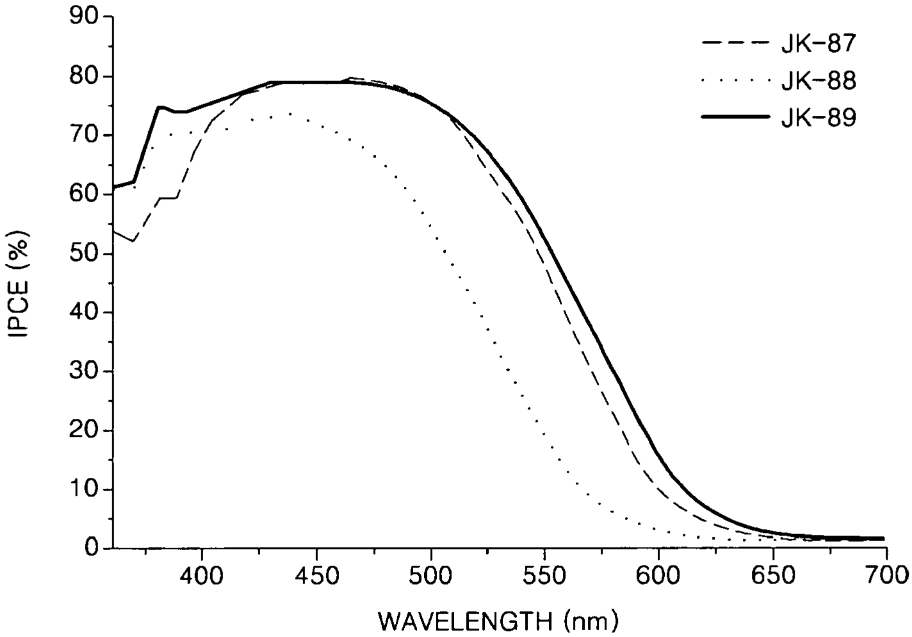

90
80
70
60
50
40
30
20
10
0
IPCE (%)
400
450
500
550
600
650
700
WAVELENGTH (nm)
JK-87
JK-88
JK-89

FIG. 3

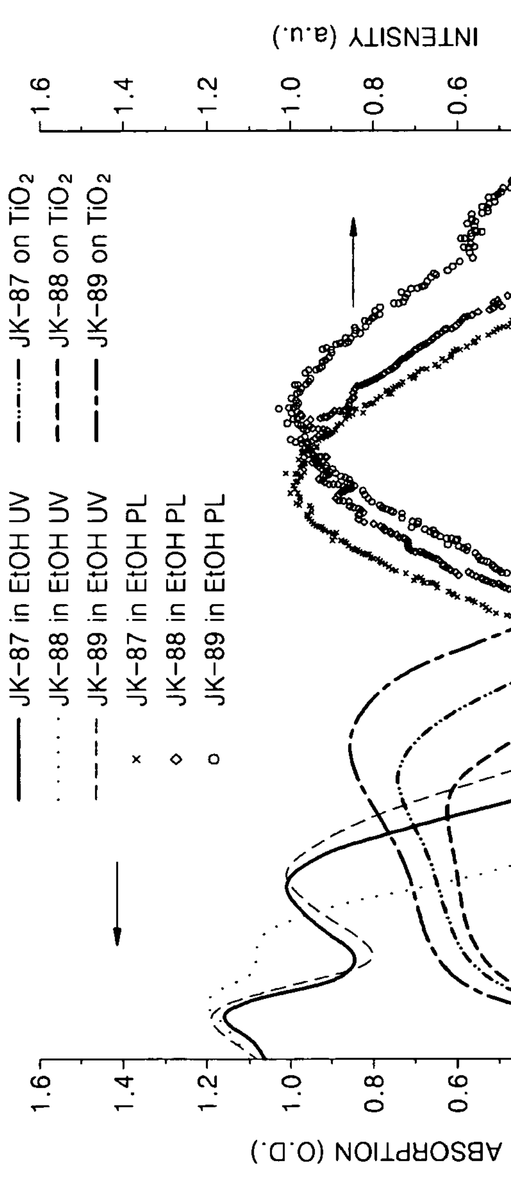
JK-87 in EtOH UV
JK-88 in EtOH UV
JK-89 in EtOH UV
JK-87 in EtOH PL
JK-88 in EtOH PL
JK-89 in EtOH PL
JK-87 on $TiO_2$
JK-88 on $TiO_2$
JK-89 on $TiO_2$
ABSORPTION (O.D.)
INTENSITY (a.u.)
WAVELENGTH (nm)
0.0
0.2
0.4
0.6
0.8
1.0
1.2
1.4
1.6
400
500
600
700

Europäisches Patentamt
European Patent Office
Office européen des brevets

# EUROPEAN SEARCH REPORT

Application Number
EP 11 25 0180

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHO, NARA ET AL: "Novel organic sensitizers containing a bulky spirobifluorene unit for solar cells", TETRAHEDRON , 65(31), 6236-6243 CODEN: TETRAB; ISSN: 0040-4020, 2009, XP002634165, * abstract * * introduction * * page 6236, compounds JK-87, JK-88, JK-89 * * figures 1,4-7 * ----- | 1-15 | INV. C07C255/42 C07D333/24 H01L31/04 H01M14/00 |
| A | DATABASE CA [Online] CHEMICAL ABSTRACTS SERVICE, COLUMBUS, OHIO, US; MOCHIZUKI, FUMITAKA ET AL: "Dye-sensitized solar cells having high durability to heat and light and high photocarrier generation efficiency", XP002634166, retrieved from STN Database accession no. 2008:977730 * abstract * -& JP 2008 186717 A (KONICA MINOLTA BUSINESS TECHNOLOGIES, INC., JAPAN) 14 August 2008 (2008-08-14) * paragraph [0060] - paragraph [0067] * * paragraph [0157] * ----- | 1,15 | TECHNICAL FIELDS SEARCHED (IPC): C07C C07D H01L H01M |

The present search report has been drawn up for all claims

1

| Place of search | Date of completion of the search | Examiner |
|---|---|---|
| The Hague | 27 April 2011 | Fitz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 25 0180

This annex lists the patent family members relating to the patent documents cited in the above-mentioned European search report.
The members are as contained in the European Patent Office EDP file on
The European Patent Office is in no way liable for these particulars which are merely given for the purpose of information.

27-04-2011

| Patent document cited in search report | Publication date | Patent family member(s) | Publication date |
| --- | --- | --- | --- |
| JP 2008186717 A | 14-08-2008 | NONE | |

EPO FORM P0459

For more details about this annex : see Official Journal of the European Patent Office, No. 12/82